(19) Europäisches Patentamt
European Patent Office
Office européen des brevets

(11) Publication number: **0 113 568**
**B1**

(12) # EUROPEAN PATENT SPECIFICATION

(45) Date of publication of patent specification: **04.04.90**

(51) Int. Cl.$^5$: **C 07 D 501/46**

(21) Application number: **83307675.5**

(22) Date of filing: **16.12.83**

(54) **Intermediates for the preparation of ceftazidine, and process for their preparation.**

(30) Priority: **27.12.82 US 453445**

(43) Date of publication of application:
**18.07.84 Bulletin 84/29**

(45) Publication of the grant of the patent:
**04.04.90 Bulletin 90/14**

(84) Designated Contracting States:
**BE CH DE FR IT LI LU NL SE**

(56) References cited:
**US-A-4 258 041**

**The file contains technical information submitted after the application was filed and not included in this specification**

(73) Proprietor: **ELI LILLY AND COMPANY**
**307, East McCarty Street**
**Indianapolis Indiana 46285 (US)**

(72) Inventor: **Chou, Ta-Sen**
**745 Canyon Road**
**Indianapolis Indiana 46217 (US)**
Inventor: **Heath, Perry Clark**
**7130 S.Delaware Street**
**Indianapolis Indiana 46227 (US)**

(74) Representative: **Crowther, Terence Roger et al**
**Erl Wood Manor**
**Windlesham Surrey GU20 6PH (GB)**

**EP 0 113 568 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a novel process and intermediate for the preparation of ceftazidime.

There is a continual need to develop new antibiotics because of the constant threat that antibiotic-specific resistant strains of pathogenic microorganisms will emerge. Because of high development and production costs, particularly in the area of cephalosporin antibiotics, researchers are searching constantly for new and efficient means for producing these antibiotics.

In this regard, U.S. Patent No. 4,258,041 describes the synthesis of (6R,7R) - 7 - [(Z) - 2 - (2 - aminothiazol - 4 - yl) - 2 - (2 - carboxyprop - 2 - oxyimino)acetamido] - 3 - (1-pyridiniummethyl)ceph - 3 - em - 4 - carboxylate, a cephalosporin antibiotic now generically known as ceftazidime. The reported synthesis of ceftazidime comprises reacting (Z) - 2 - (2 - *tert.* - butoxycarbonylprop - 2 - oxyimino) - 2 - (2 - triphenylmethylaminothiazol - 4 - yl) - acetyl chloride with (6R,7R) - 7 - amino - 3 - (1 - pyridiniummethyl)ceph - 3 - em - 4 - carboxylic acid in a mixture of N,N-dimethylacetamide and acetonitrile to provide the ceftazidime intermediate, (6R,7R) - 7 - [(Z) - 2 - (2 - triphenylmethylamino-thiazol - 4 - yl) - 2 - (2 - *tert.* - butoxycarbonylprop - 2 - oxyimino)acetamido] - 3 - (1 - pyridinium-methyl) - ceph - 3 - em - 4 - carboxylate. This ceftazidime intermediate then was recovered from the reaction mixture as an amorphous solid and subsequently purified by crystallization as an N,N-dimethyl-formamide solvate (2½ moles of DMF per mole of intermediate) from N,N-dimethylformamide. The ceftazidime intermediate then was reacted with formic acid to effect removal of the protecting groups and to provide ceftazidime.

The present invention provides an improved process for preparing the ceftazidime intermediate, in which the intermediate is crystallized directly out of the reaction mixture as an N,N-dimethylacetamide solvate. This invention, therefore, obviates the need for subsequent purification and provides improved yields and reduced costs in the production of ceftazidime.

In accordance with the invention, a crystalline compound of Formula (I):

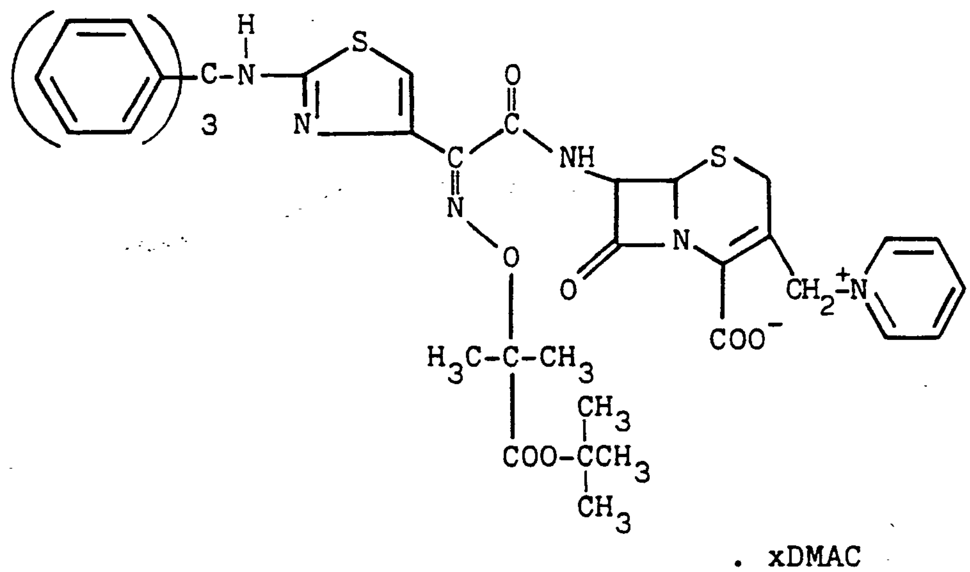

(I)

in which DMAC is N,N-dimethylacetamide and x is a number from 0.5 to 3.0, is a useful intermediate in the synthesis of ceftazidime.

Also, there is provided a process for preparing a compound of Formula (I), as defined above, which comprises reacting in N,N-dimethylacetamide a ((Z)-2-(2-*tert.*-butoxycarbonylprop-2-oxyimino)-2-(2-triphenylmethylamino-thiazol-4-yl)acetyl halide with a (6R,7R)-7-amino-3-(1-pyridiniummethyl)ceph-3-em-4-carboxylic acid salt. The resulting dimethylacetamide solvate can be isolated as a crystalline solid directly from the reaction mixture. The process of this invention allows one to isolate, in good yield, substantially pure ceftazidime intermediate N,N-dimethylacetamide solvate.

As used throughout this description, "ceftazidime intermediate" means (6R,7R) - 7 - [(Z) - 2 - (2 - triphenylmethylaminothiazol - 4 - yl) - 2 - (2 - *tert.* - butoxycarbonylprop - 2 - oxyimino)acetamido] - 3 - (1 - pyridiniummethyl)ceph - 3 - em - 4 - carboxylate. The process by which this ceftazidime intermediate is prepared involves a typical acylation reaction comprising reacting a cephalosporin nucleus (i.e. a 7-amino-3-cephem derivative) with an acid halide, typically an acid chloride. The acid halide typically employed in the synthesis of the ceftazidime intermediate is a (Z)-2-(2-*tert.*-butoxycarbonylprop-2-oxyimino)-2-(2-triphenylmethylaminothiazol-4-yl)acetic acid halide. The prior art acylation reaction

generally is accomplished by reacting the acid halide with about an equimolar quantity of the 7-amino-3-cephem nucleus in an unreactive solvent such as dichloromethane, acetone, or a mixture of N,N-dimethylacetamide and acetonitrile, and in the presence of a base such as triethylamine or pyridine. The present process is improved by employing N,N-dimethylacetamide as the reaction solvent thereby producing the ceftazidime intermediate directly from the acylation reaction mixture as a crystalline N,N-dimethylacetamide solvate. This improved process eliminates the separate purification step in which ceftazidime intermediate is converted to a solvate such as the N,N-dimethylformamide solvate.

In carrying out the process of this invention, the acid halide, for instance (Z)-2-(2-*tert.*-butoxycarbonylprop-2-oxyimino)-2-(2-triphenylmethylaminothiazol-4-yl)acetyl chloride, may be prepared by reaction of the free acid with a halogenating agent such as phosphorus pentachloride in a suitable solvent such as dichloromethane or diethyl ether. Once the acid halide is formed, it normally is not isolated but rather is added to a suspension of about an equimolor quantity of the cephalosporin nucleus in N,N-dimethylacetamide. A suitable base such as triethylamine is added to the reaction mixture to serve as scavenger for any free acid that might be present. The acylation reaction typically is complete within about fifteen to ninety minutes when carried out at a temperature of about −20 to about 40°C. The ceftazidine intermediate that is thus produced is an N,N-dimethylacetamide solvate of Formula (I):

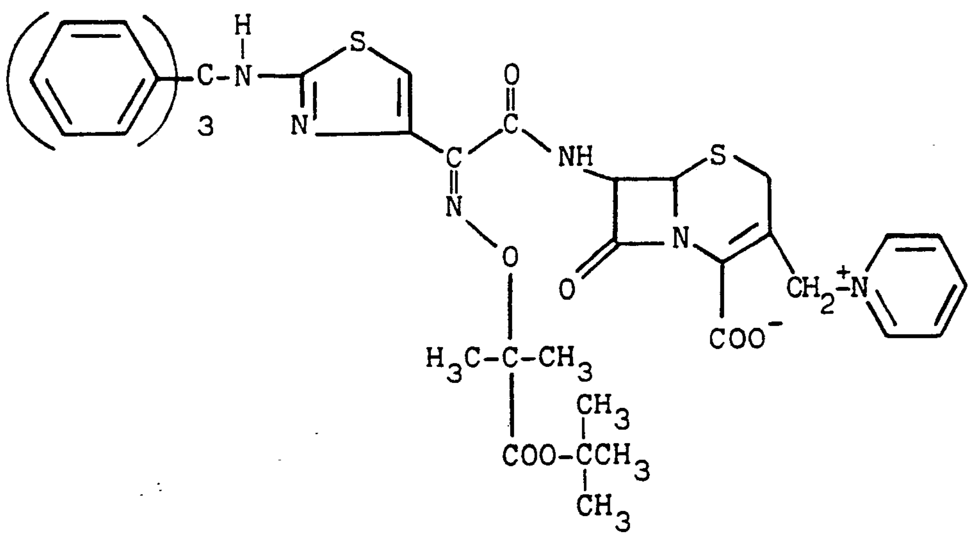

(I)

. xDMAC

in which DMAC is N,N-dimethylacetamide and x is a number from 0.5 to 3.0 and denotes the number of moles of DMAC per mole of ceftazidime intermediate. Typically solvates may be those in which x is 0.5, 1.0, 1.5, 2.0 or 2.5. Preferred solvates are those in which x is about 0.5 to about 1.5, and especially in which x is 1.0.

The ceftazidime intermediate solvate can be isolated readily if desired, by washing the reaction mixture with water and then adding a suitable counter solvent, i.e. a solvent in which the intermediate is substantially insoluble. Typical counter solvents include ethers, such as diethyl ether, methyl ethyl ether, diglyme, or tetrahydrofuran; and esters such as ethyl acetate, or methyl acetate. The counter solvent of choice is diethyl ether, or a combination of diethyl ether and ethyl acetate.

When the counter solvent is added to the N,N-dimethylacetamide reaction mixture, the ceftazidime intermediate N,N-dimethylacetamide solvate starts to precipitate within about thirty to about ninety minutes as the solution is stirred at about −10 to about +10°C. The crystalline solvate is isolated by simply filtering the mixture, and generally, washing the solvate with fresh N,N-dimethylacetamide or diethyl ether is desirable. The product can be air dried at room temperature or dried in vacuum to remove any excess solvent. The product produced is an N,N-dimethylacetamide solvate of ceftazidime intermediate having a purity generally greater than about ninety percent.

The ceftazidime intermediate solvate provided by this invention may be employed directly to produce ceftazidime. For example, the crystalline solvate may be dissolved in an acid solution, such as 98% formic acid, to cleave the triphenylmethyl protecting group on the amino group attached to the thiazolylacetamido side chain, and also to cleave the *tert.*-butoxy protecting group on the oxime portion of the side chain. If desired, hydrochloric acid can be added so as to form the dihydrochloride salt of ceftazidime, thereby facilitating isolation of the final product as a crystalline precipitate. The ceftazidime dihydrochloride produced can be employed as an antibiotic.

To further illustrate the invention the following examples are provided.

Example 1

(6R,7R)-7-[(Z)-2-(2-triphenylmethylaminothiazol-4-yl)-2-(2-*tert.*-butoxycarbonylprop-2-oxyimino)-acetamido]-3-(1-pyridiniummethyl)ceph-3-em-4-carboxylate DMAC solvate.

To a cold (−10°C) stirred solution of 8.70 g (41.8 mM) of phosphorus pentachloride in 150 ml of dichloromethane were added in one portion 21.72 g (38 mM) of (Z)-2-(2-*tert.*-butoxycarbonylprop-2-oxyimino)-2-(2-triphenylmethylaminothiazol-4-yl)acetic acid. The reaction mixture was stirred for thirty minutes at −10°C, and then diluted with a cold solution of 100 ml of water containing 11.66 ml (83.6 mM) of triethylamine. The two-phase reaction mixture was stirred vigorously for about three minutes, and then the organic layer was removed and added to a stirred cold (−10°C) suspension of 16.89 g (38.0 mM) of (6R,7R)-7-amino-3-(1-pyridiniummethyl)ceph-3-em-4-carboxylic acid dihydrochloride in 195 ml of N,N-dimethylacetamide containing 26.5 ml (190 mM) of triethylamine. The reaction mixture was stirred at 0 to −5°C for thirty minutes, and then was diluted by the addition in one portion of 300 ml of water. The aqueous reaction mixture was stirred for ten minutes, and then the organic layer was separated and further diluted by the addition of 150 ml of fresh N,N-dimethylacetamide and 300 ml of diethyl ether. The organic solution was stirred at 0 to 5°C for one hour, and the crystalline precipitate that had formed was collected by filtration, washed with fresh N,N-dimethylacetamide and then with fresh diethyl ether, and was dried at ambient temperature in vacuum for sixteen hours to produce 20.96 g (65.2% yield) of (6R,7R) - 7 - [(Z) - 2 - (2-triphenylmethylaminothiazol - 4 - yl) - 2 - (2 - *tert.* - butoxycarbonylprop - 2 - oxyimino)acetamido] - 3 - (1 - pyridiniummethyl)ceph - 3 - em - 4 - carboxylate N,N - dimethylacetamide solvate (1 mole of N,N-dimethylacetamide), m.p. 150°C (dec). Purity as determined by high performance liquid chromatography was 95.56%.

Example 2

The process of Example 1 was repeated as follows:

To a cold (−15°C) stirred solution of 7.73 g of phosphorus pentachloride in 120 ml of dichloromethane were added in one portion 17.32 g of (Z)-2-(2-*tert.*-butoxycarbonylprop-2-oxyimino)-2-(2-triphenylmethylaminothiazol-4-yl)acetic acid. The reaction mixture was stirred for thirty minutes while maintaining the temperature at about −10 to −15°C. The reaction mixture was then diluted by the addition of 80 ml of water containing 10.5 ml of triethylamine. After stirring the reaction mixture for three minutes, the organic layer was separated and the aqueous layer was discarded. The organic layer was then added dropwise over ten minutes to a cold (−10°C) stirred suspension of 11.13 g of (6R,7R)-7-amino-3-(1-pyridiniummethyl)-ceph-3-em-4-carboxylic acid dihydrochloride in 88 ml of N,N-dimethylacetamide containing 17.5 ml of triethylamine. The reaction mixture was stirred for thirty minutes at about −5 to 0°C, and then was allowed to warm to about +5°C and was diluted by the addition of 200 ml of water. After stirring the aqueous mixture for two minutes, the organic layer was separated and the aqueous layer was discarded. The organic layer was further diluted with 200 ml of ethyl acetate and 100 ml of diethyl ether, and the mixture was vigorously stirred at about 20 to 25°C. The mixture was seeded with ceftazidime intermediate N,N-dimethylacetamide solvate to initiate crystallization. After stirring the mixture for thirty minutes at 20 to 25°C, the temperature was lowered to 0°C and stirring was continued for about two hours. Filtration of the mixture and washing of the filter cake with a mixture of 10 ml of N,N-dimethylacetamide and 10 ml of diethyl ether and finally with 40 ml of diethyl ether provided, following drying at 35°C under vacuum for about sixteen hours, about 78 percent yield of ceftazidime intermediate N,N-dimethylacetamide solvate (1.5 mole of N,N-dimethylacetamide).

Example 3

Ceftazidime Dihydrochloride

To a 250 ml, 3-necked, round-bottom flask purged with nitrogen are added 62.2 ml (75.9 g) of formic acid which is cooled to about 15°C. To the stirring cooled formic acid is added 32.0 g (37.9 mM) of the ceftazidime intermediate from Example 1 to 2. The solution is stirred for 30 minutes after which 13.3 ml (15.8 g) of hydrochloric acid are added while maintaining the temperature below 20°C. The mixture is stirred for about 3 hours and the triphenylmethanol produced is filtered and washed with formic acid and water. The filtrate is cooled and added to about 850 ml of acetone after which the cooled slurry is stirred for about 2 hours. The product is filtered and washed with acetone to produce the title product in about a 62% yield.

NMR (DMSO-$d_6$): δ 1.58 (s, 2—$CH_3$), δ 5.33 (d, J=5Hz, 6—H), δ 6.01 (dd, J=9.5Hz, 7—H), δ 7.00 (s, aminothiazole), δ 8.30, 8.75 (t, J=6Hz), δ 8.75 (t, J=6Hz), δ 9.23 (d, J=6Hz, pyridinium protons), δ 9.72 (d, J=9, acetamido proton).

**Claims**

1. A crystalline compound of Formula (1):

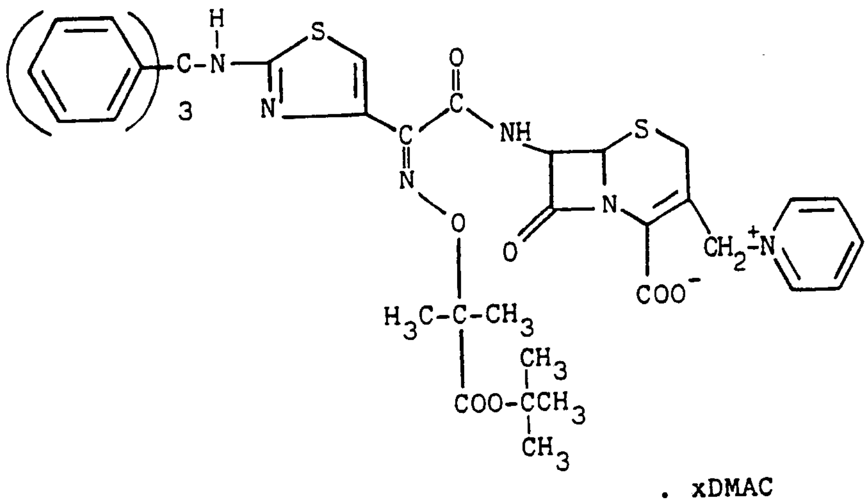

(I)

. xDMAC

in which DMAC is N,N-dimethylacetamide and x is a number from 0.5 to 3.0.

2. A compound of Formula (I) as claimed in claim 1 in which x is 0.5.

3. A compound of Formula (I) as claimed in claim 1 in which x is 1.0.

4. A compound of Formula (I) as claimed in claim 1 in which x is 1.5.

5. A compound of Formula (I) as claimed in claim 1 in which x is 2.0.

6. A compound of Formula (I) as claimed in claim 1 in which x is 2.5.

7. A process for preparing a compound of Formula (I) as claimed in any one of claims 1 to 6 comprising reacting in N,N-dimethylacetamide a ((Z)-2-(2-*tert.*-butoxycarbonylprop-2-oxyimino)-2-(2-triphenylmethyl-amino-thiazol-4-yl))acetyl halide with a (6R,7R)-7-amino-3-(1-pyridiniummethyl)ceph-3-em-4-carboxylic acid salt, characterised in that after formation of the product, the reaction mixture is washed with water and a countersolvent is added to produce a crystalline solvate.

8. A process for preparing ceftazidime or a pharmaceutically acceptable salt thereof, which comprises removing the protecting groups from a compound of Formula (1) as claimed in any one of claims 1 to 6.

9. A process as claimed in claim 8 in which the protecting groups are removed by treating with acid the compound for Formula (I).

10. A compound of Formula (I) as claimed in any one of claims 1 to 6 for use as an intermediate in the preparation of ceftazidime.

**Patentansprüche**

1. Kristalline Verbindung der Formel (I)

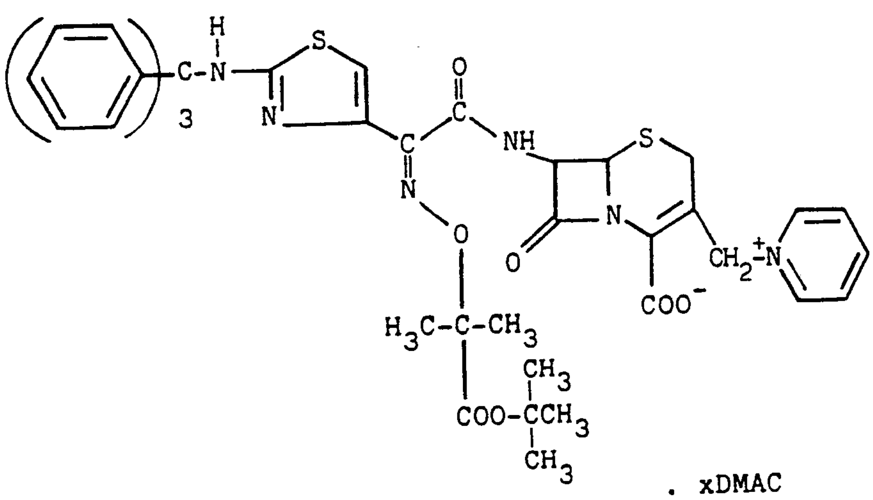

(I)

. xDMAC

worin DMAC für N,N-Dimethylacetamid steht und x eine Zahl von 0,5 bis 3,0 ist.

2. Verbindung der Formel (I) nach Anspruch 1, dadurch gekennzeichnet, daß x für 0,5 steht.
3. Verbindung der Formel (I) nach Anspruch 1, dadurch gekennzeichnet, daß x für 1,0 steht.
4. Verbindung der Formel (I) nach Anspruch 1, dadurch gekennzeichnet, daß x für 1,5 steht.
5. Verbindung der Formel (I) nach Anspruch 1, dadurch gekennzeichnet, daß x für 2,0 steht.
6. Verbindung der Formel (I) nach Anspruch 1, dadurch gekennzeichnet, daß x für 2,5 steht.
7. Verfahren zur Herstellung einer Verbindung der Formel (I) nach einem der Ansprüche 1 bis 6, durch Umsetzung eines ((Z) - 2 - (2 - t - Butoxycarbonylprop - 2 - oxyimino) - 2 - (2 - triphenylmethylaminothiazol - 4 - yl))acetylhalogenids mit einem (6R,7R) - 7 - Amino - 3 - (1 - pyridiniummethyl)ceph-3-em-4-carbonsäuresalz in N,N-Dimethylacetamid, dadurch gekennzeichnet, daß das Reaktionsgemisch nach der Bildung des Produkts mit Wasser gewaschen wird und daß zur Bildung eines kristallinen Solvats ein Gegenlösungsmittel zugesetzt wird.
8. Verfahren zur Herstellung von Ceftazidim oder einem pharmazeutisch annehmbaren Salz hiervon, dadurch gekennzeichnet, daß von einer Verbindung der Formel (I) gemäß einem der Ansprüche 1 bis 6 die Schutzgruppen entfernt werden.
9. Verfahren nach Anspruch 8, dadurch gekennzeichnet, daß die Schutzgruppen durch Behandlung der Verbindung der Formel (I) mit einer Säure entfernt werden.
10. Verbindung der Formel (I) nach einem der Ansprüche 1 bis 6 zur Verwendung als Zwischenprodukt für die Herstellung von Ceftazidim.

**Revendications**

1. Composé cristallin de formule (I):

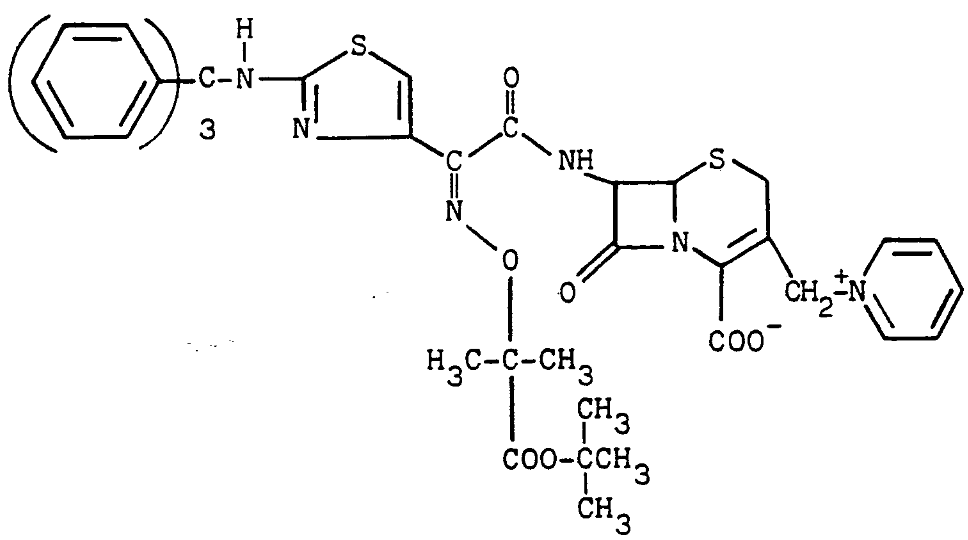

(I)

. xDMAC

dans laquelle DMAC représente le N,N-diméthylacétamide et x est un nombre de 0,5 à 3,0.
2. Composé de formule (I) selon la revendication 1, formule dans laquelle x représente 0,5.
3. Composé de formule (I) selon la revendication 1, formule dans laquelle x représente 1,0.
4. Composé de formule (I) selon la revendication 1, formule dans laquelle x représente 1,5.
5. Composé de formule (I) selon la revendication 1, formule dans laquelle x représente 2,0.
6. Composé de formule (I) selon la revendication 1, formule dans laquelle x représente 2,5.
7. Procédé de préparation d'un composé de formule (I) selon l'une quelconque des revendications 1 à 6, ce procédé consistant à faire réagir, dans du N,N-diméthylacétamide, un halogénure de ((Z) - 2 - (2 - *tert.* - butoxycarbonylprop - 2 - oxyimino) - 2 - (2 - triphénylméthylamino - thiazol - 4 - yl)) - acétyle avec un sel d'acide (6R,7R) - 7 - amino - 3 - (1 - pyridiniumméthyl) - céph - 3 - em - 4 - carboxylique, caractérisé en ce que, après la formation du produit, on lave le mélange réactionnel avec de l'eau et on ajoute un contre-solvant pour obtenir un produit de solvatation cristallin.
8. Procédé de préparation de ceftazidime ou d'un de ses sels pharmaceutiquement acceptables, caractérisé en ce qu'il consiste à éliminer les groupes protecteurs d'un composé de formule (I) selon l'une quelconque des revendications 1 à 6.
9. Procédé selon la revendication 8, caractérisé en ce qu'on élimine les groupes protecteurs en traitant, avec un acide, le composé de formule (I).
10. Composé de formule (I) selon l'une quelconque des revendications 1 à 6, en vue de l'utiliser comme produit intermédiaire lors de la préparation du ceftazidime.